# EUROPÄISCHE PATENTSCHRIFT

(11) **EP 1 774 922 B1**
(45) Veröffentlichungstag und Bekanntmachung des Hinweises auf die Patenterteilung: **11.06.2008**
(21) Anmeldenummer: 05022214.0
(22) Anmeldetag: 12.10.2005
(51) Int. Cl.: A61B 19/00

(54) **Marker für ein Navigationssystem und Verfahren zum Detektieren eines Markers**
Marker for a navigation system and method for detecting a marker
Marqueur pour un système de navigation et procédé de detection d'un marqueur

(43) Veröffentlichungstag der Anmeldung: 18.04.2007
(73) Patentinhaber: BrainLAB AG, 85551 Kirchheim/Heimstetten (DE)
(72) Erfinder: Blumhofer, Andreas, Dr., 85579 Neubiberg (DE); Manus, Johannes, Dr., 81479 München (DE)
(74) Vertreter: Schwabe - Sandmair - Marx

(56) Entgegenhaltungen:
- DE-A1- 10 306 793
- DE-U1-7202004 011 56
- US-A1- 2002 188 194
- US-A1- 2004 167 393
- US-B1- 6 675 040

## Beschreibung

Die vorliegende Erfindung bezieht sich auf einen Marker und insbesondere auf eine Markeranordnung, wie sie insbesondere für ein Navigationssystem zur Bestimmung der räumlichen Position zum Beispiel eines chirurgischen Instrumentes geeignet ist, sowie auf ein Verfahren zum Detektieren der Position eines Markers.

Marker werden verwendet, um zum Beispiel bei bildunterstützten chirurgischen Verfahren (IGS; Image Guided Surgery) die Position von mit zum Beispiel mehreren Markern in Form eines Referenzsternes verbundenen chirurgischen Instrumenten oder Körpern durch eine optische Erfassung des von den Markern reflektierten Lichtes zu ermitteln und basierend auf den ermittelten Positionsinformationen einen chirurgischen Eingriff durchzuführen.

Das Reflexionsvermögen von kugelförmigen passiven Markern ist nicht homogen, da die auf die Kugeloberfläche aufgebrachte retro-reflektive Folie oder eine Beschichtung nur innerhalb eines bestimmten Winkelbereiches ein gutes Reflexionsvermögen aufweist. Jedoch ist der Grenzwinkel für das Reflexionsvermögen nicht mit hoher Genauigkeit reproduzierbar, so dass das Reflexionsvermögen am sichtbaren Rand eines Markers nicht definiert bestimmbar ist. Die Inhomogenität des Reflexionsvermögens eines Markers wird zusätzliche durch das Aufbringen der Folie auf die kugelförmige Basis verstärkt, wodurch es zu nicht definierten Verzerrungen der reflektierenden Folie kommt. Demzufolge ist die Genauigkeit der Positionsbestimmung eines Tracking-Systems begrenzt, insbesondere wenn Marker-Erkennungs-Algorithmen verwendet werden, welche auf der Detektion des Massenmittelpunktes basieren.

Werden flache Marker verwendet, so kann, wenn der Massenmittelpunkt der Reflexion eines zum Beispiel schräg zur Erfassungsebene liegenden kreisförmigen reflektierenden Markers detektiert wird, eine Positionsungenauigkeit dadurch zustande kommen, dass der kreisförmige in die Kameraebene projizierte reflektierende Marker eine Ellipse ist, deren Massenmittelpunkt nicht exakt beim Mittelpunkt des kreisförmigen Markers liegt. Weiterhin können Ungenauigkeiten beim Aufbringen der Folie auftreten.

Aus der GB-2,404,453 A ist ein kugelförmiger retro-reflektiver Videomarker bekannt, welcher an einem Objekt für ein Video-Tracking befestigt werden kann.

Aus DE 20 2004 011 567 U1 ist ein Reflektorelement für einen Marker eines chirurgischen Navigationssystems mit einer flächigen Reflektorschicht bekannt, die auf einem Träger aufliegt und die im Bereich einer definierten Fläche reflektierend wirkt, wobei der Träger in dem Auflagebereich der Reflektorschicht eine Ausdehnung aufweist, die der definierten Fläche entspricht und wobei die Reflektorschicht als Stanzteil ausgebildet ist, dessen Aussenkante längs des äußeren Randes des Trägers im Anlagebereich verläuft.

Es ist eine Aufgabe der vorliegenden Erfindung einen Marker, welcher kostengünstig hergestellt werden kann und eine sichere Positionsdetektion ermöglicht, sowie ein Verfahren zum sicheren Detektieren eines solchen Markers vorzuschlagen.

Ein erfindungsgemäßer Marker oder eine Markeranordnung, insbesondere zur Verwendung in Verbindung mit einem Navigationssystem, weist eine Licht reflektierende zum Beispiel eben oder flach ausgebildete oder eine oder mehrere Krümmungen aufweisende Oberfläche auf, wobei ein Licht absorbierendes oder intransparentes oder opakes Objekt in einem definierten Abstand von z.B. 0, 0.5, 1.0 oder mehr als 1.0 mm oder auch mit einem variablen Abstand zur reflektierenden Oberfläche angebracht ist. Das Licht absorbierende oder nicht-reflektierende Objekt ist eine Kugel oder Kugelförmig und ist mit der reflektierenden Oberfläche vorzugsweise fest verbunden, zum Beispiel mittels eines Abstandhalters oder Stabes, welcher zum Beispiel an einem definierten Punkt der reflektierenden Oberfläche, wie zum Beispiel in der Mitte der reflektierenden Oberfläche, angeordnet sein kann und zum Beispiel auf den Mittelpunkt einer als absorbierendes Objekt dienenden Kugel gerichtet ist.

Mit einem erfindungsgemäßen Marker oder einer Markeranordnung kann eine höhere Genauigkeit bei der Positionserkennung erzielt werden, da zum Beispiel im Falle der Verwendung einer Kugel als Licht absorbierendes Objekt das von einer Kamera erfassbare Markerbild ein exakter Kreis mit scharfem und gut erkennbarem Umriss ist und somit zuverlässigere Kreisdetektionsalgorithmen verwendet werden können. Weiterhin besitzt das Marker-Bild einen hohen Kontrast, d.h. eine scharfe Kontur beim Übergang zwischen einer von einer Kamera erkannten reflektierenden Fläche und der durch das absorbierende Objekt abgeschirmten reflektierenden Fläche, welche z.B. als dunkle Fläche erkannt werden kann. Allgemein kann erfindungsgemäß eine reflektierende Fläche auch mit einem Körper verwendet werden, welcher ein anderes und z.B. schlechteres Reflexionsvermögen als eine bei dem Körper angeordnete reflektierende Fläche hat, wobei von einem Navigationssystem die unterschiedlichen Helligkeiten oder Lichtstärken der erfassten zweidimensionalen Objekt- und Reflexionsflächenbilder erkannt und ausgewertet werden.

Demgegenüber kann mit bekannten Markern, insbesondere bei mit Folien bezogenen oder beschichteten Markern, kein starker Kontrast realisiert werden.

Weiterhin ist bei Verwendung des erfindungsgemäßen Markers das von einer Kamera erfassbare Marker-Bild bei gleicher Größe der Marker-Kugel größer, da die im Sichtfeld der Erfassungskamera liegende reflektierende Fläche von der Kugel vollständig abgedeckt wird, wohingegen eine mit einer reflektierenden Substanz beschichtete Marker-Kugel im Randbereich keine volle Reflexion mehr erzeugt und somit ein relativ kleineres Marker-Bild liefert. Da bei der erfindungsgemäßen Markeranordnung das reflektierende Objekt, wie zum Beispiel eine Kugel, nicht mit einer retro-reflektierenden Folie beschichtet sein muss, kann diese auch für weitere Zwecke verwendet werden, wie zum Beispiel als Marker für die Positionsbestimmung mittels eines Lasertrackers (High Precision Laser Range Tracker) oder ein mechanisches Koordinaten-Messgerät.

Da es erfindungsgemäß möglich ist eine ebene reflektierende Oberfläche zu verwenden, können die Produktionskosten für einen Marker verringert werden.

Vorzugsweise ist die reflektierende Oberfläche retro-reflektiv und weist zum Beispiel eine kreisförmige oder rechteckige, wie zum Beispiel quadratische, Oberfläche auf, wobei zum Beispiel im Kreismittelpunkt oder im Schwerpunkt der reflektierenden Oberfläche ein an diesem Punkt z.B. senkrecht von der reflektierenden Oberfläche herausragendes Befestigungselement für das absorbierende Objekt angebracht ist.

Vorteilhaft werden mindestens zwei und zum Beispiel drei oder mehr wie oben beschriebene Marker verwendet, welche beispielsweise als Referenzstern dienen können. Dabei können über einer einzigen oder mehreren unterschiedlichen reflektierenden Oberflächen zwei, drei oder mehr nicht oder schwach reflektierenden oder Licht absorbierende Objekte mit gleicher oder verschiedener Geometrie mit gleichem oder verschiedenem Abstand zur reflektierenden Oberfläche angeordnet werden.

Gemäß einem weiteren Aspekt bezieht sich die Erfindung auf ein optisches z.B. passives Navigationssystem mit bevorzugt mindestens einer Lichtquelle zur Emission von Licht, wobei unter Licht im Sinne der Erfindung jede Form elektro-magnetischer Wellen verstanden werden kann, wie zum Beispiel Licht im sichtbaren oder Infrarot-Bereich. Weiterhin weist das Navigationssystem mindestens eine wie oben beschriebene Markeranordnung auf, wobei von mindestens einer Kamera das von der Lichtquelle emittierte und von der reflektierenden Oberfläche-reflektierte-Licht-erfasst wird. Die Kamera ist mit einer Recheneinheit verbunden, welche die von der Kamera erfassten optischen Signale auswertet und den durch das oberhalb der reflektierenden Oberfläche im Sichtbereich der Kamera liegende absorbierende Objekt erzeugten dunklen oder nicht-reflektierenden Bereich innerhalb der reflektierenden Oberfläche erkennt und hieraus z.B. in Verbindung mit der Information über die Markeranordnung oder Geometrie der Markeranordnung, d.h. z.B. der bekannten in einer Datenbank gespeicherten Information über die Form des absorbierenden Objektes und dessen Abstand von der reflektierenden Oberfläche, die Position des Markers oder der Markeranordnung oder eines Referenzsternes im Raum berechnet.

Gemäß einem weiteren Aspekt bezieht sich die Erfindung auf ein Verfahren zum Detektieren einer wie oben beschriebenen Markeranordnung, wobei ermittelt wird, ob ein von einer Kamera erfasstes Bild oder Teilbild der reflektierenden Oberfläche ein absorbierendes die Oberfläche zumindest zum Teil abschirmendes Objekt aufweist und überprüft wird, ob der nur schwach oder nicht-reflektierende Bereich vollständig von einem reflektierenden Bereich umgeben wird oder nicht. Falls der nicht-reflektierende Bereich vollständig von einem reflektierenden Bereich umgeben wird, so kann die Position des absorbierenden Objektes zum Beispiel durch Bestimmung des Mittelpunktes eines Kreises im Falle der Verwendung einer Kugel als absorbierendes Objekt bestimmt werden. Falls der nicht-reflektierende Bereich nicht vollständig von einem reflektierenden Bereich umgeben wird, so kann erkannt werden, dass sich aus dem Blickwinkel der Erfassungskamera gesehen das absorbierende Objekt nicht mehr vollständig vor einer reflektierenden darunter liegenden Oberfläche befindet, so dass diese Erfassungsdaten der Kamera als fehlerhaft erkannt werden können. Wird somit bei dem erfindungsgemäßen Verfahren festgestellt, dass das Abbild des absorbierenden Objektes aus der reflektierenden Oberfläche herauswandert, so kann ein Fehler detektiert werden, wodurch das Fehlerdektektionsrisiko, also die falsche Bestimmung der Position eines Markers, ausgeschlossen werden kann.

Vorzugsweise wird das erfindungsgemäße Verfahren zum Kalibrieren zum Beispiel einer Kamera, eines Navigationssystems, eines medizinischen Instrumentes oder eines Patienten verwendet, mit welchem die Marker verbunden sind, wobei die Marker nur relativ wenig bewegt werden.

Gemäß einem weitern Aspekt bezieht sich die Erfindung auf ein Computerprogramm, welches, wenn es in einen Computer geladen ist oder auf einem Computer läuft, das oben beschriebene Verfahren durchführt. Ebenso bezieht sich die Erfindung auf ein Programmspeichermedium oder Computerprogrammprodukt mit einem solchen Programm.

Die Erfindung wird nachfolgend anhand eines Ausführungsbeispieles beschrieben.

Es zeigt:

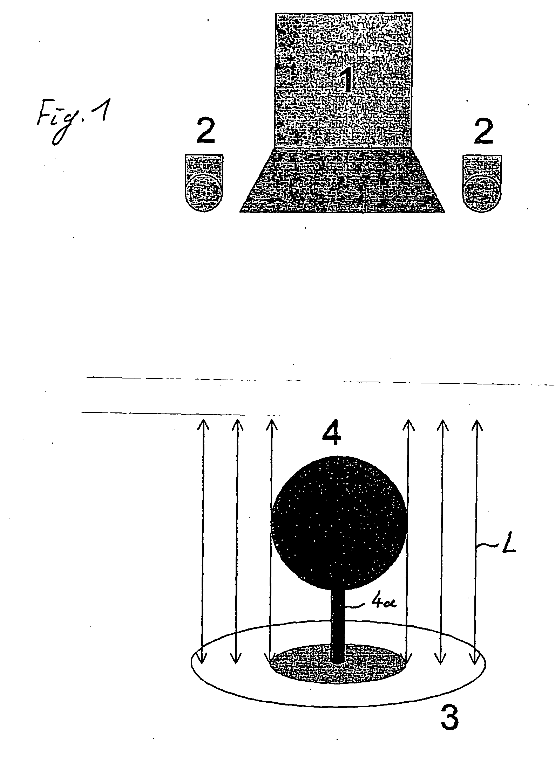

- Figur 1: ein Navigationssystem zur Erfassung eines erfindungsgemäßen Markers.

Figur 1 zeigt eine kreisförmige retro-reflektierende Fläche 3, an welcher an deren Mittelpunkt ein senkrecht von der reflektierenden Fläche 3 vorstehendes Abstandselement 4a befestigt ist, auf welchem eine als absorbierendes Objekt dienende Licht absorbierende Kugel 4 angeordnet ist. Von Lichtemittern 2 wird Licht L auf die gezeigte Markeranordnung 3, 4 ausgesandt, welches von der reflektierenden Fläche 3 nur in dem nicht von der Licht absorbierenden Kugel 4 abgedeckten Bereich reflektiert wird. Dieses Reflexionsmuster kann von einer Videokamera 1 erfasst werden, welche anhand des im gezeigten Ausführungsbeispiel ringförmigen Reflexions-Bildes erkennen kann wie die Position des Markers 3, 4 oder eines aus mehreren Markern gebildeten Referenzsterns im Raum ist.

Erfindungsgemäß wird somit neben einem Reflexionsbild auch ein von einem oder mehreren absorbierenden Objekten 4 erzeugter Reflexions-Schatten detektiert, welcher eine scharfe Abgrenzung zwischen dem reflektierenden und schwach oder nicht reflektierenden Bereich der reflektierenden Fläche 3 ermöglicht, wodurch erfindungsgemäß die Erfassungsgenauigkeit erhöht werden kann.

## Patentansprüche

1. Marker bestehend aus einer reflektierenden Oberfläche (3) und einem Licht absorbierenden oder nicht oder nur schwach reflektierenden Objekt (4), welches in einem festen oder variablen Abstand vor der reflektierenden Oberfläche (3) angeordnet ist, **dadurch gekennzeichnet, dass** das absorbierende Objekt (4) eine Kugel oder kugelförmig ist.

2. Marker nach Anspruch 1, wobei die reflektierende Oberfläche (3) eine ebene oder gekrümmte Oberfläche ist.

3. Marker nach einem der vorhergehenden Ansprüche, wobei das absorbierende Objekt (4) fest mit der reflektierenden Oberfläche mittels eines Verbindungselements (4a) verbunden ist.

4. Marker nach einem der vorhergehenden Ansprüche, wobei die reflektierende Oberfläche (3) kreisförmig, rechteckig oder quadratisch ist.

5. Marker nach einem der vorhergehenden Ansprüche, wobei die reflektierende Oberfläche (3) retro-reflektiv ist.

6. Markeranordnung mit mindestens zwei oder drei Markern nach einem der vorhergehenden Ansprüche.

7. Navigationssystem mit mindestens einem Marker (3, 4) nach einem der Ansprüche 1 bis 5 oder mindestens einer Markeranordnung nach Anspruch 6, mindestens einer Kamera (1), welche ein Reflexionsbild des mindestens einen Markers (3, 4) erfassen kann und einer Recheneinheit, welche mit der Kamera (1) verbunden ist und anhand des Reflexionsbildes des mindestens einen Markers (3, 4) die Position des Markers (3, 4) oder der Markeranordnung im Raum bestimmen kann.

8. Navigationssystem nach Anspruch 7 mit mindestens einer Lichtquelle (2) zur Beleuchtung des mindestens einen Markers (3, 4).

9. Verfahren zum Detektieren einer Position eines Markers oder einer Markeranordnung (3, 4) nach einem der Ansprüche 1 bis 6, wobei ermittelt wird, ob in dem Reflexionsbild eine Licht nicht reflektierende Fläche vollständig von einer Licht reflektierenden Fläche umgeben wird, um zu bestimmen, ob eine Fehldetektion vorliegt.

10. Verfahren nach dem vorhergehenden Anspruch, wobei das Detektionsverfahren zum Kalibrieren einer Kamera, eines Navigationssystems, eines Instruments oder Körpers verwendet wird.

11. Computerprogramm, welches, wenn es auf einem Computer läuft oder in einen Computer geladen wird, das Verfahren nach einem der beiden vorhergehenden Ansprüche durchführt.

12. Programmspeichermedium oder Computerprogrammprodukt mit dem Programm nach dem vorhergehenden Anspruch.

## Claims

1. A marker consisting of a reflective surface (3) and a light-absorbing or non-reflective or but low-reflective object (4) which is arranged at a fixed or variable distance in front of the reflective surface (3), **characterised in that** the absorbing object (4) is a sphere or is spherical.

2. The marker according to claim 1, wherein the reflective surface (3) is a plane or curved surface.

3. The marker according to any one of the preceding claims, wherein the absorbing object (4) is fixedly connected to the reflective surface, by means of a connecting element (4a).

4. The marker according to any one of the preceding claims, wherein the reflective surface (3) is circular, rectangular or square.

5. The marker according to any one of the preceding claims, wherein the reflective surface (3) is retro-reflective.

6. A marker array comprising at least two or three markers according to any one of the preceding claims.

7. A navigation system comprising at least one marker (3, 4) according to any one of claims 1 to 5 or at least one marker array according to claim 6, at least one camera (1) which can detect a reflection image of the at least one marker (3, 4), and a computational unit which is connected to the camera (1) and can determine the spatial position of the marker (3, 4) or marker array on the basis of the reflection image of the at least one marker (3, 4).

8. The navigation system according to claim 7, comprising at least one light source (2) for illuminating the at least one marker (3, 4).

9. A method for detecting a position of a marker or marker array (3, 4) according to any one of claims 1 to 6, which ascertains whether an area which does not reflect light is completely surrounded by a light-reflective area in the reflection image, in order to determine whether a detection is incorrect.

10. The method according to the preceding claim, wherein the detection method is used to calibrate a camera, a navigation system, an instrument or a body.

11. A computer program which, when running on a computer or loaded onto a computer, performs the method according to any one of the preceding two claims.

12. A program storage medium or computer program product comprising the program according to the preceding claim.

## Revendications

1. Repère constitué d'une surface réfléchissante (3) et d'un objet (4) absorbant la lumière ou ne la réfléchissant pas ou faiblement seulement, lequel est disposé à une distance fixe ou variable devant la surface réfléchissante (3), **caractérisé en ce que** l'objet absorbant (4) est une sphère ou est en forme de sphère.

2. Repère selon la revendication 1 dans lequel la surface réfléchissante (3) est une surface plane ou courbe.

3. Repère selon l'une des revendications 1 ou 2, dans lequel l'objet réfléchissant (4) est relié de manière fixe à la surface réfléchissante au moyen d'un élément de liaison (4a).

4. Repère selon l'une quelconque des revendications 1 à 3, dans lequel la surface réfléchissante (3) est de forme circulaire, rectangulaire ou carrée.

5. Repère selon l'une quelconque des revendications 1 à 4, dans lequel la surface réfléchissante (3) est rétroréfléchissante.

6. Agencement de repères avec au moins deux ou trois repères selon l'une quelconque des revendications 1 à 5.

7. Système de navigation avec au moins un repère (3, 4) selon l'une quelconque des revendications 1 à 5 ou au moins un agencement de repères selon la revendication 6, au moins une caméra (5) qui peut capter une image réfléchie du au moins un repère (3, 4), et une unité informatique qui est reliée à la caméra (1), et la position du repère (3, 4) ou de l'agencement de repères dans l'espace peut être déterminée à l'aide de l'image réfléchie du au moins un repère (3, 4).

8. Système de navigation selon la revendication 7 avec au moins une source de lumière (2) pour éclairer le au moins un repère (3, 4).

9. Procédé de détection d'une position d'un repère ou d'un agencement de repères (3, 4) selon l'une quelconque des revendications 1 à 6, dans lequel il est déterminé si, dans l'image réfléchie, une surface ne réfléchissant pas la lumière est entièrement entourée par une surface réfléchissant la lumière, afin de déterminer si l'on est en présence d'une erreur de détection.

10. Procédé selon la revendication précédente, dans lequel le procédé de détection est utilisé pour étalonner une caméra d'un système de navigation, d'un instrument ou d'un corps.

11. Programme d'ordinateur qui, lorsqu'il tourne sur un ordinateur ou est chargé dans un ordinateur, exécute le procédé selon l'une des deux revendications précédentes.

12. Support de mémoire de programme ou produit de programme d'ordinateur avec le programme selon la revendication précédente.
